(19) Europäisches Patentamt / European Patent Office / Office européen des brevets

(11) **EP 3 425 556 A1**

(12) **EUROPEAN PATENT APPLICATION**
published in accordance with Art. 153(4) EPC

(43) Date of publication:
**09.01.2019 Bulletin 2019/02**

(51) Int Cl.:
***G06K 9/00*** (2006.01)

(21) Application number: **17783694.7**

(22) Date of filing: **03.05.2017**

(86) International application number:
**PCT/CN2017/082917**

(87) International publication number:
**WO 2018/201350 (08.11.2018 Gazette 2018/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Shenzhen Goodix Technology Co., Ltd.**
**Shenzhen, Guangdong 518045 (CN)**

(72) Inventors:
• **PANG, Shu**
**Shenzhen**
**Guangdong 518045 (CN)**
• **LIANG, Jianhua**
**Shenzhen**
**Guangdong 518045 (CN)**
• **ZHONG, Zhixin**
**Shenzhen**
**Guangdong 518045 (CN)**
• **ZHONG, Hua**
**Shenzhen**
**Guangdong 518045 (CN)**

(74) Representative: **Valea AB**
**Box 7086**
**103 87 Stockholm (SE)**

(54) **VITAL SIGN INFORMATION DETERMINATION METHOD, IDENTITY AUTHENTICATION METHOD AND DEVICE**

(57) Embodiments of the present application provide a vital sign information determination method, and an identity authentication method and apparatus. The vital sign information determination method includes: acquiring first biological feature data collected when an object presses on a biological feature sensor, the first biological feature data being used to reflect a body texture feature of the object; parsing a plurality of pieces of first biological feature data of the object to acquire second biological feature data, the second biological feature data being used to reflect a vital sign of a body of the object; and determining vital sign information of the body of the object according to the second biological feature data. On the prerequisite of not changing the structure of a conventional biological feature sensor and configuring an additional aliveness identification sensor, the vital sign information may be conveniently identified by using the same biological feature sensor, which solves the problem that the implementation cost is high due to additional configuration of the aliveness identification sensor when the aliveness identification function is added, and further implements identity authentication based on aliveness identification.

```
┌─────────────────────────────────────┐
│ Acquire first biological feature     │──── S11
│ collected when an object presses on  │
│ a biological feature sensor          │
└─────────────────────────────────────┘
                  │
                  ▼
┌─────────────────────────────────────┐
│ Parse a plurality of first biological│──── S12
│ feature data of the object to acquire│
│ second biological feature data       │
└─────────────────────────────────────┘
                  │
                  ▼
┌─────────────────────────────────────┐
│ Determine vital sign information of  │──── S13
│ the body of the object according to  │
│ the second biological feature data   │
└─────────────────────────────────────┘
```

FIG. 1

## Description

### TECHNICAL FIELD

Embodiments of the present application relate to the technical field of identity authentication, and in particular, relate to a method for determining vital sign information, an identity authentication method and apparatus.

### BACKGROUND

With introduction of the fingerprint identification technology to mobile terminals, fingerprint-based unlocking has replaced password-based unlocking, slide-unlocking and the like. In this way, unlocking does not need other operations, but may be simply implemented by a finger in contact with a sensor. This greatly improves use convenience of the mobile terminals while ensuring security of the mobile terminals.

However, mobile terminals generally store a large amount of personal information, which involves security of properties and privacies of users. After the mobile terminals employ fingerprint identification, unauthorized users may steal fingerprints of the users to fabricate spoof fingerprints, and crack security systems of the users to obtain user information in the mobile terminals. This adversely increases the probability that the fingerprint passwords of the mobile terminals are cracked, and causes greater threatens to information security of the mobile terminals.

Accordingly, in the prior art, aliveness identification is performed by configuring an independent aliveness identification sensor, that is, the aliveness identification sensor is provided in addition to the fingerprint sensor. However, this solution needs two independent sensors, which increases the implementation cost.

### SUMMARY

Embodiments of the present application are intended to provide a vital sign information determination method, an identity authentication method and apparatus, to at least solve the above technical problem in the prior art.

Embodiments of the present application provide a vital sign information determination method. The method includes:

acquiring first biological feature data collected when an object presses on a biological feature sensor, the first biological feature data being used to reflect a body texture feature of the object;

parsing a plurality of pieces of first biological feature data of the object to acquire second biological feature data, the second biological feature data being used to reflect a vital sign of a body of the object; and

determining vital sign information of the body of the object according to the second biological feature data.

Embodiments of the present application provide an identity authentication method. The method includes:

acquiring first biological feature data collected when an object presses on a biological feature sensor, and performing identity authentication for the object according to the first biological feature data, the first biological feature data being used to reflect a body texture feature of the object;

parsing a plurality of pieces of first biological feature data of the object to acquire second biological feature data, determining vital sign information of the body of the object according to the second biological feature data, and performing aliveness identification for the object according to the vital sign information, the second biological feature data being used to reflect a vital sign of a body of the object; and

judging whether the identity authentication is successful according to a result of the identity authentication and a result of the aliveness identification.

Embodiments of the present application provide a biological feature sensor. The biological feature sensor includes: a light source, a sensing array, a biological feature chip. Light emitted by the light source is reflected by an object and received by the sensing array to generate first biological feature data; the biological feature chip is configured to acquire the first biological feature data collected when the object presses on the biological feature sensor; the first biological feature data is parsed to determine vital signal information of a body of the object; where the first biological feature data is used to reflect a body texture feature of the object, and the second biological feature data is used to reflect a vital sign of the body of the object.

Embodiments of the present application provide an electronic terminal. The electronic terminal includes the biological feature sensor as described in any of the above embodiments.

In embodiments of the present application, first biological feature data collected when an object presses on a biological feature sensor is acquired, where the first biological feature data is used to reflect a body texture feature of the object; a plurality of pieces of first biological feature data of the object is parsed to acquire second biological feature data, where the second biological feature data is used to reflect a vital sign of a body of the object; and vital sign information of the body of the object is determined according to the second biological feature data. On the prerequisite of not changing the structure of a conventional biological feature sensor and configuring an additional aliveness identification sensor, the vital sign information may be conveniently identified by using the same biological feature sensor, which solves the problem that the implementation cost is high due to additional configuration of the aliveness identification sensor when the aliveness identification function is added, and further implements identity authentication based on aliveness identification.

## BRIEF DESCRIPTION OF THE DRAWINGS

FIG. 1 is a schematic flowchart of a method for determining vital sign information according to an embodiment of the present application;

FIG. 2a is a schematic structural diagram of a biological feature sensor according to an embodiment of the present application;

FIG. 2b is a schematic structural diagram of a sensing unit in the biological feature sensor in FIG. 2a;

FIG. 3 is a waveform diagram obtained according to fingerprint feature data according to an embodiment of the present application;

FIG. 4 is a waveform diagram obtained according to fingerprint feature data according to an embodiment of the present application;

FIG. 5a is a schematic flowchart of an identity authentication method according to an embodiment of the present application;

FIG. 5b is a schematic diagram of a sequence of scanning sensing units in the identity authentication method in FIG. 5a;

FIG. 6a is a schematic flowchart of a method for determining heart rate information according to an embodiment of the present application; and

FIG. 6b is a schematic diagram of a scanning region of sensing units in the heart rate information determination method in FIG. 6a.

## DETAILED DESCRIPTION

Practice of the present application is described in detail with reference to drawings and specific embodiments, such that the practice of addressing the technical problem using the technical means according to the present application and achieving the technical effects may be better understood and conducted.

In the embodiments of the present application hereinafter, description is given using a case that a biological feature sensor is a fingerprint feature sensor for collecting fingerprint feature data, second biological feature data is heart rate data and vital sign information is heart rate information as an example. It should be noted that, in other embodiments, the vital sign information may also be other biological feature data, such as blood oxygen, blood pressure, or the like.

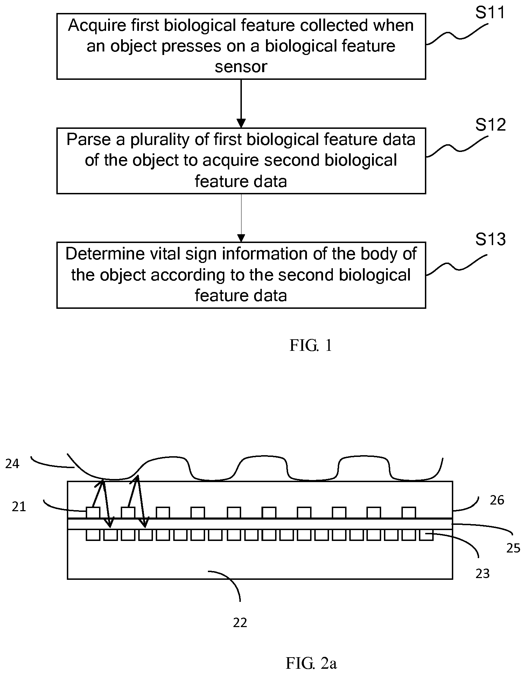

FIG. 1 is a schematic flowchart of a method for determining vital sign information according to an embodiment of the present application. As illustrated in FIG. 1, the method includes the following steps:

S11: First biological feature data, which is collected when an object presses on a biological feature sensor, is acquired, where the first biological feature data is used to reflect a body texture feature of the object.

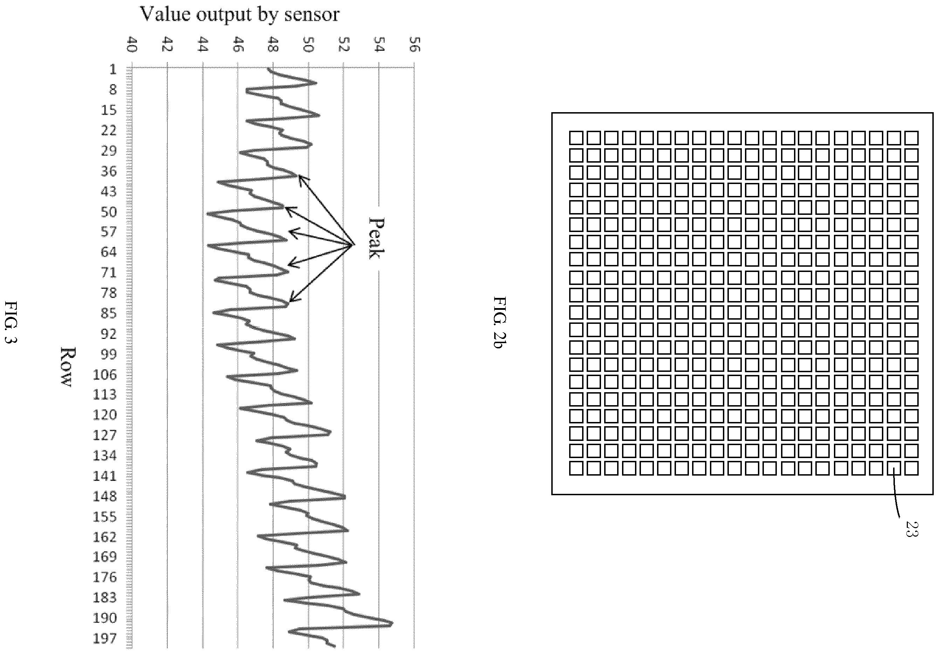

In this embodiment, the object may be specifically a finger, a palm or the like. The biological feature sensor may be a sensor which operates based on light reflection principle. As illustrated in FIG. 2a and FIG. 2b, the biological feature sensor may specifically includes a light source 21, a sensing array (not illustrated in the drawings) and a biological feature chip 22; the sensing array may include a plurality of sensing units 23. When the object presses on the biological feature sensor, light emitted by the light source is reflected by the object (for example, a finger 24) and then received by the sensing units 23 in the sensing array. Each sensing unit in the sensing array may generate a piece of first biological feature data, and thus a plurality of pieces of first biological feature data are generated. The biological feature chip acquires the plurality of pieces of first biological feature data of the object, which are collected when the object presses on the biological feature sensor, and parses the plurality of pieces of first biological feature data of the object to determine vital sign information of the object. As illustrated in FIG. 2a, the biological feature sensor may further include an optical filter 25. The optical filter has a filter function, for example, a function of filtering interference light contained in the light reflected by the object.

Specifically, the light emitted by the light source is irradiated on the object, reflected by the object and then received by the sensing array; the sensing array converts a reflected optical signal into an electrical signal. Valley and ridge textures of a fingerprint or a palmprint result in different reflections for the light. For example, an optical signal reflected by the valley has a greater strength and the corresponding electrical signal is also greater, and an optical reflected by the ridge has a smaller strength and the corresponding electrical signal is also smaller. Therefore, based on formation of such differentiated electrical signals, the plurality of pieces of first biological feature data, which reflects a texture feature of the object, may be acquired.

In this embodiment, specifically, the light source may share an organic light-emitting diode (OLED) light source of an OLED display screen 26, such that the biological feature sensor may be directly applied to the OLED display screen, thereby forming an in-display structure. In other embodiments, the light source may also be an independent light source.

In addition, the sensing array may be arranged below the OLED display screen to form an under-display structure.

In this embodiment, the plurality of pieces of first biological feature data collected when the object presses on the biological feature sensor, which reflects a texture feature of the object, may be specifically a plurality of pieces of first biological feature data of the object that are collected when the object presses on a predetermined region of the biological feature sensor. The size of the predetermined region may be flexibly defined according to actual needs. For example, the position of the predetermined region may be defined in a setting option of an electronic terminal.

In this embodiment, the acquiring the plurality of pieces of first biological feature data collected when the object presses on the biological feature sensor, which reflects the texture feature of the object, may specifically include: when the object presses on the biological feature sensor, acquiring the plurality of pieces of first biological feature data that are collected according to a predetermined collection rule.

In this embodiment, the predetermined collection rule may be determined according to an arrangement manner of sensing arrays on the biological feature sensor. For example, as illustrated in FIG. 2b, if the sensing array includes a plurality of sensing units that are arranged in row and column directions, the predetermined collection rule may be scanning the sensing units in the row direction or in the column direction to collect the plurality of pieces of first biological feature data.

S12: The plurality of pieces of first biological feature data of the object are parsed to acquire second biological feature data, where the second biological feature data is used to reflect a vital sign of a body of the object.

In this embodiment, as described above, if an object presses on the sensing array, a plurality of first biological feature data may be generated. However, if the object is a live body, when the light emitted by the light source is irradiated on the object, a portion of the emitted light may be directly reflected, and another portion of the emitted light may be transmitted through the skin tissue and then be reflected. Therefore, in this course, some light may be absorbed by the skin tissue, thus the optical signal obtained upon reflection may be subjected to attenuation. Absorption of the light by the muscles, bones and the like in the skin tissue is fixed, and absorption of the light by the blood is variable due to blood flowing. Specifically, different flowing speeds of the blood cause different flow volumes of the blood tissue, and thus absorption of the light by the blood is variable. As such, the optical signal obtained via reflection after the optical signal is transmitted through the skin tissue is subjected to different attenuations, and the optical signals received by the sensing array are also subjected to different attenuations, such that the plurality of pieces of first biological feature data not only includes information reflecting blood flow volume variations, but also includes the texture information reflecting the object. Therefore, the second biological feature data reflecting the vital sign of the body of the object may be determined by parsing the plurality of pieces of first biological feature data of the object, for example, heartbeat data (that is, the heart rate data) affecting the blood flow volume may be determined.

Specifically, in this embodiment, the plurality of pieces of first biological feature data of the object may be parsed to remove the texture information therefrom, such that the second biological feature data of the body of the object is obtained and the vital signal information of the body of the object is acquired.

In this embodiment, when the plurality of pieces of first biological feature data are acquired, the plurality of pieces of first biological feature data of the object may be grouped according to the predetermined collection rule, and then the grouped pieces of first biological feature data are parsed to acquire the second biological feature data of the body of the object.

Exemplarily, as described above, assume that the object is a finger, the first biological feature data is fingerprint data, the second biological feature data is heart rate data, and the vital sign information is heart rate information; the biological feature sensor includes m rows*n columns of sensing units. During data collection, the sensing units may be scanned according to a predetermined scanning rule to acquire a plurality of pieces of fingerprint feature data of the finger. For example, the sensing units are scanned in the row direction or in the column direction to collect the fingerprint feature data. During grouping, the fingerprint feature data may be grouped according to the predetermined collection rule in the row direction or column direction.

Exemplary grouping is described as follow. if the fingerprint feature data is one frame of fingerprint feature data collected within one cycle on the sensing array of the biological feature sensor, grouping the fingerprint feature data of the finger according to the predetermined collection rule (for example, scanning in the row direction or in the column direction) includes: grouping the fingerprint feature data of the finger according to a collection rule predetermined within one cycle. For example, the fingerprint feature data is grouped into m groups (grouping based on row) or n groups (grouping based on column), that is, a plurality of pieces of first biological feature data obtained by performing one row scanning cycle for the sensing array or performing one column scanning cycle for the sensing array are used as a group of first biological feature data.

In another embodiment, if the fingerprint feature data is multiple frames of fingerprint feature data collected within multiple cycles on the sensing array of the biological feature sensor, grouping the fingerprint feature data includes: using one frame of fingerprint feature data as a group of fingerprint feature data.

As compared with the one-frame circumstance, in the multi-frame circumstance, since the sample amount of the fingerprint feature data is great, the accuracy of the heart rate data determined in the multi-frame circumstance is higher.

Specifically, step S12 includes the predetermined collection rule; if the first biological feature data is fingerprint feature data, in this step, the fingerprint feature data is parsed upon grouping to remove the texture information from the fingerprint feature data to acquire the heart rate data, which may specifically include: calculating an average value of the plurality of pieces of fingerprint feature data in different groups to remove the texture information therefrom respectively, to acquire a plurality of pieces of heart rate data. Each group of fingerprint feature data corresponds to a respective piece of heart rate data.

Specifically, in this embodiment or other embodiments, if the sensing array includes m rows * n columns of sensing units, the fingerprint feature data may be collected by scanning the sensing units in the row direction. In this case, the obtained plurality of pieces of fingerprint feature data may be grouped according to rows, and the plurality of pieces of fingerprint feature data obtained by performing one scanning cycle for the sensing array are used as a group of fingerprint feature data. That is, upon grouping, m groups of fingerprint feature data in total may be obtained, and each group of fingerprint feature data includes n pieces of fingerprint feature data. An average value of each group of fingerprint feature data may be calculated using the method shown in formula (1) as follows:

$$A_r = \frac{\sum\limits_{e=1}^{n} D_{r,e}}{n} \quad (1)$$

$D_{r,e}$ represents fingerprint feature data in the $r^{th}$ row and the $e^{th}$ column, n represents the total number of columns in the sensing array, and $A_r$ represents an average value of the fingerprint feature data in the $r^{th}$ row, $1 \leq r \leq m$, $1 \leq e \leq n$.

In other embodiments, if the fingerprint feature data is grouped according to columns, the method for calculating an average is the same as the method for calculating the average of the fingerprint feature data upon grouping according to rows, which is not described herein any further.

Alternatively, in this embodiment or other embodiments, if data collection is performed for the predetermined region of the sensing array, assuming that the predetermined region includes a rows and b columns of sensing units of the sensing array, one frame of fingerprint feature data is formed after the a rows and b columns of sensing array are scanned, and c frames of fingerprint feature data are obtained by performing scanning for multiple times for the a rows and b columns of sensing units. In this case, each frame of first biological feature data may be used as a respective group of first biological feature data, and upon grouping c groups of fingerprint feature data in total may be obtained. Each group of fingerprint feature data includes a*b pieces of fingerprint feature data. An average value of each group of fingerprint feature data is calculated using the method shown in formula (2) as follows:

$$A_f = \frac{\sum\limits_{r=g}^{g+a} \sum\limits_{e=h}^{h+b} D_{r,e}}{a*b}, \quad (2)$$

$D_{r,e}$ represents fingerprint feature data in the $r^{th}$ row and the $e^{th}$ column, g and g+a respectively represent a starting row and an ending row in the predetermined region, h and h+b respectively represent a starting column and an ending column in the predetermined region (corresponding to a portion of or all of the sensing arrays), and $A_f$ represents an average value of the $f^{th}$ frame of fingerprint feature data, $g \leq r \leq g+a$, $h \leq e \leq h+b$, $1 \leq f \leq c$.

It should be noted that the predetermined region may correspond to a portion of the sensing array, or may correspond to the entire sensing array.

S13: Vital sign information of the body of the object is determined according to the second biological feature data.

In this embodiment, considering that the acquired heart rate data may include noise, step S13 further includes filtering the heart rate data, and then determining the heart rate information of the body of the finger according to the heart rate data upon filtering. Specifically, the filtering may include average value filtering, Gaussian filtering and the like, and the method for filtering the heart rate data to remove noise included in the data may be specifically referenced to other known methods.

In this embodiment, when the heart rate information of the body of the finger is determined according to the heart rate data, a waveform peak statistical collection or spectrum analysis may be performed for the heart rate data of the body of the finger to determine the heart rate information of the body of the finger. Specifically, in case that the fingerprint feature data is grouped, the waveform peak value statistical collection or spectrum analysis may be performed for average values of the different groups of fingerprint feature data, to determine the heart rate information of the body of the finger.

Specifically, when the fingerprint feature data is one frame of fingerprint feature data collected within one cycle on the sensing array of the biological feature sensor, performing the waveform peak statistical collection or spectrum analysis for the data to determine the heart rate information of the body of the finger includes: performing the waveform peak statistical collection or spectrum analysis for a plurality of pieces of heart rate data, which are obtained by respectively parsing several groups of fingerprint feature data formed by grouping one frame of fingerprint feature data, to determine the heart rate information of the body of the finger.

Alternatively, in other embodiments, when the fingerprint feature data of the finger is multiple frames of fingerprint feature data collected within multiple cycles on the sensing array of the biological feature sensor, performing waveform peak statistical collection or spectrum analysis for the heart rate data to determine the heart rate information of the body of the finger includes: performing the waveform peak statistical collection or spectrum analysis for a plurality of pieces of heart rate data, which are obtained by respectively parsing several groups of fingerprint feature data formed by grouping multiple frames of fingerprint feature data, to determine the heart rate information of the body of the finger.

Specifically, hereinafter performing the waveform peak statistical collection for the heart rate data to determine the heart rate information of the body of the finger is described. Firstly, a schematic waveform is drawn according to the average value of each group of fingerprint feature data, and then the heart rate information (that is, the heart rate value) is determined according to formula (3) as follows:

$$H=M/T, (3)$$

M represents the number of peaks in the waveform, T represents a total scanning time elapsed when one frame or multiple frames of fingerprint feature data are obtained, H represents a frequency corresponding to the peak, that is, the heart rate information (the heart rate value). A schematic waveform is as illustrated in FIG. 3. FIG. 3 is a waveform obtained by processing the fingerprint feature data after one frame of fingerprint feature is collected by scanning in the row direction.

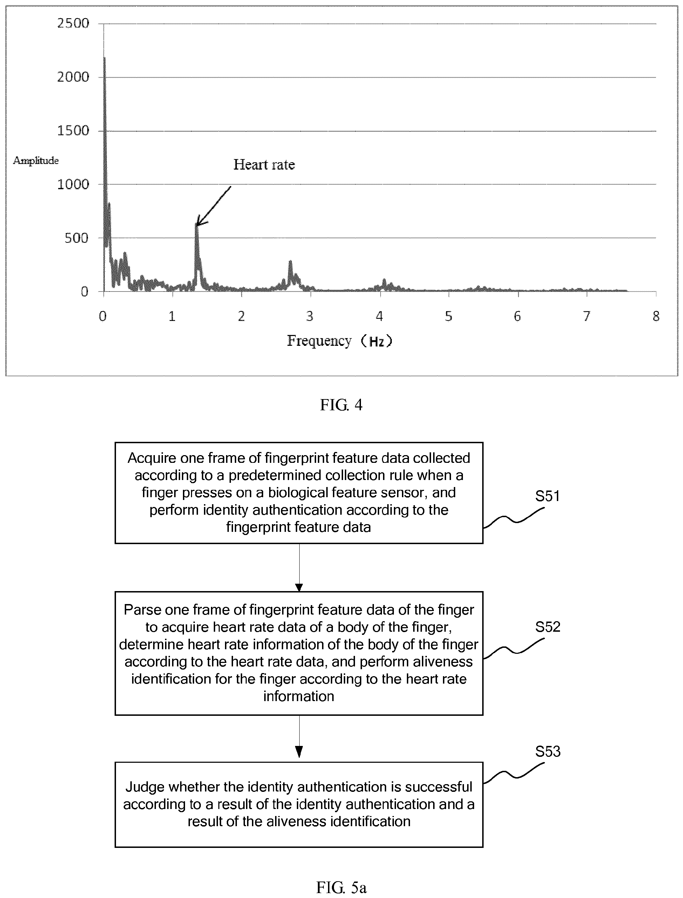

Hereinafter description is given using performing spectrum analysis to determine heart rate information of a body of the finger as an example. An average value of each group of fingerprint feature data is subjected to a Fourier transformation to obtain the corresponding spectrum data. In combination with the fact that in normal circumstances the heart rate range of the human body is 60 to 100 beats/minute (which may be approximately equal to 1 Hz), the frequency corresponding to a maximum amplitude in the vicinity of the frequency of 1 Hz in the spectrum data is determined as the heart rate information. An exemplary waveform is as illustrated in FIG. 4.

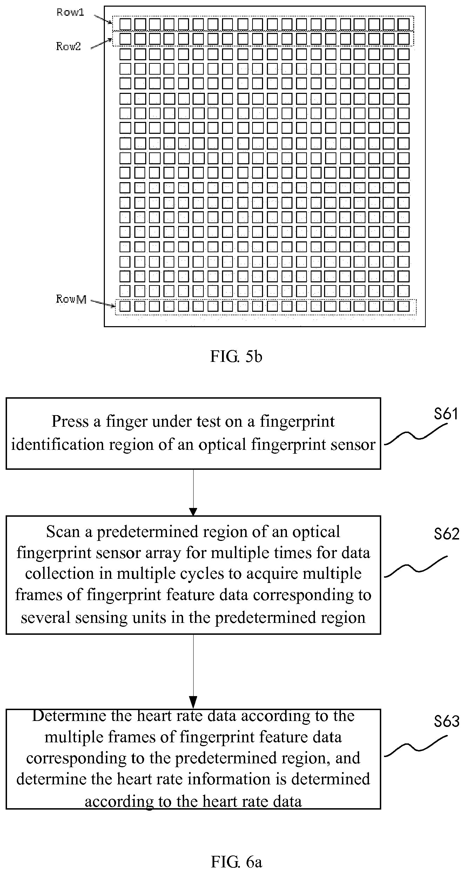

FIG. 5 is a schematic flowchart of an identity authentication method according to an embodiment of the present application. As illustrated in FIG. 5, in this embodiment, description is given hereinafter still using the case that the object is a finger, the first biological feature data is fingerprint feature data, the biological feature sensor is an optical fingerprint sensor, the second biological feature data is heart rate data, and the vital sign information is heart rate information as an example. After identity authentication is implemented using one frame of fingerprint feature data, the vital sign information is acquired, which specifically includes the following steps.

S51: One frame of fingerprint feature data collected according to a predetermined collection rule when a finger press on a biological feature sensor is acquired, and identity authentication is performed according to the fingerprint feature data.

The predetermined collection rule may be scanning all the sensing units according to rows (which may also be based on columns). As illustrated in FIG. 5b, the sensor firstly scans row 1, then scans row 2,..., and finally scans row m. The frequency for scanning the sensing units is a predetermined scanning frequency, such that any two neighboring rows are scanned with a same time interval. This time interval may be flexibly defined. Specifically, assuming that the heart rate has a maximum value of 2.6 Hz, , the predetermined scanning frequency is greater than 2*2.6 Hz according to the sampling theory; that is, the time interval is less than 1/(2*2.6 Hz)s. Therefore, the less the time interval is, the more accurate the obtained data is.

Upon completion of data collection, the obtained fingerprint feature data is processed to obtain texture information of the fingerprint, and thus the identity authentication may be performed according to the texture information of the fingerprint. The specific method for performing identity authentication according to the texture information of the fingerprint may be referenced to other known methods.

S52: One frame of fingerprint feature data of the finger is parsed to acquire heart rate data of a body of the finger, heart rate information of the body of the finger is determined according to the heart rate data, and hence aliveness identification is performed for the finger according to the heart rate information.

The specific heart rate information determination method may be referenced to the above embodiment, which is not described herein any further.

Specifically, after the heart rate information is determined, the heart rate information may be compared with pre-stored reference heart rate information to perform aliveness identification to the object.

Specifically, in a specific application environment, when the finger with a fingerprint glove is contact with and pressed on the biological feature sensor, since the fingerprint glove between the finger and the biological feature sensor may hinder transmission of the light, the light transmitted to skin tissue and the light reflected by the skin tissue are both reduced, such that data corresponding to the vital sign information in the obtained fingerprint feature data of the finger is less. As a result, the determined heart rate information is not within the range of the pre-stored reference heart rate information. Therefore, in this step, by the comparison of the determined heart rate information and the pre-stored reference heart rate information, if the determined heart rate information is within the range of the pre-stored reference heart rate information, it may be determined that the collected fingerprint feature data is from a live body, and otherwise, it may be determined that the fingerprint feature data is not collected from a live body. The range of the pre-stored reference heart rate information may be customized according to individual differences.

S53: Whether the identity authentication is successful is judged according to a result of the identity authentication and a result of the aliveness identification.

Specifically, if the identity authentication is successful, that is, the texture information included in the collected fingerprint feature data is consistent with pre-stored texture information, and in addition, if the aliveness identification is successful, that is, the heart rate information included in the collected fingerprint feature data is within the range of the pre-stored reference heart information, it may be determined that the identity authentication is successful, and otherwise, it may be determined that the identity authentication is unsuccessful.

During the aliveness identification, a requirement of the accuracy of the vital sign information is low, and it is only required that the vital sign information is within the range of pre-stored reference vital sign information. Therefore, in this embodiment, a sample amount of the fingerprint feature data obtained by scanning one frame of fingerprint feature data is small, and thus the speed of collecting fingerprint feature data may be improved. In addition, it is not required that the finger is in contact with and pressed on the optical fingerprint sensor for long time. In this way, on the prerequisite of not affecting user experience, an aliveness identification function is added to the conventional biological feature sensor, and security of the identity authentication is improved.

It may be understood that, in the embodiment of the present application, performing identity authentication according to the fingerprint feature data and performing aliveness identification according to the heart rate information are not subjected to a fixed sequence, and the sequence of performing identity authentication and performing aliveness identification is not limited in this embodiment.

In another practice of this embodiment, the identity authentication may be performed for the finger according to all or a part of the fingerprint feature data when the heart rate data of the body of the finger is acquired. Specifically, multiple frames of fingerprint feature data may be collected by scanning all the sensing units, for example, m rows and n columns of sensing units, of the optical sensor. In this case, the heart rate information may be determined according to the collected multiple frames of fingerprint feature data, and the identity authentication may be performed for the object according to all or a part of the fingerprint feature data collected when the object presses on the biological feature sensor. For example, the identity authentication is performed according to one frame of fingerprint feature data in the collected multiple frames of fingerprint feature data; or the identity authentication is performed according to the collected multiple frames of fingerprint feature data. If it is determined that the heart rate information uses the multiple frames of fingerprint feature data, as compared with collecting only one frame of fingerprint feature data, the sample amount of the fingerprint feature data is increased, and thus the determined heart rate information is more accurate.

FIG. 6a is a schematic flowchart of a method for determining heart rate information according to an embodiment of the present application. In this embodiment, as illustrated in FIG. 6a, the method includes the following steps:

S61: A finger under test press on a fingerprint identification region of an optical fingerprint sensor.

S62: A predetermined region of an optical fingerprint sensor array is scanned for multiple times for data collection in multiple cycles, to acquire multiple frames of fingerprint feature data corresponding to a plurality of sensing units in the predetermined region.

In this embodiment, the sensing units corresponding to the predetermined region are a portion of all the sensing units. The sensing units in the predetermined region are scanned, rather than all the sensing units in the sensing array, such that a sample amount of the acquired first biological feature data is reduced.

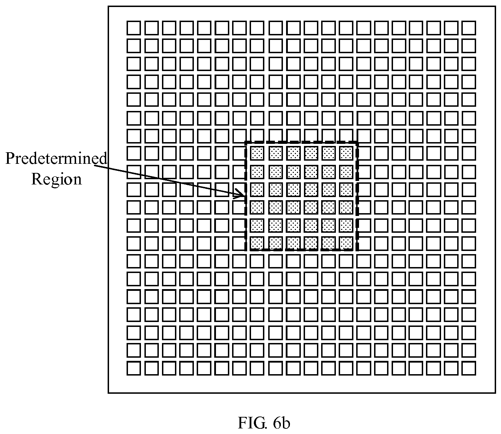

Specifically, in this embodiment, as illustrated in FIG. 6b, the predetermined region may be a small region in the middle of the sensing array. During scanning of the sensing array, to ensure accuracy of the heart rate information, the sensing units may be scanned at a predetermined scanning frequency, such that any two neighboring frames are scanned with a same time interval. This time interval may be defined. Specifically, assuming that the heart rate has a maximum value of 2.6 Hz, the predetermined scanning frequency is greater than 2*2.6 Hz based on the sampling theory; that is, the time interval between scannings of two neighboring frames is less than 1/(2*2.6 Hz)s.

S63: The heart rate data is determined according to the multiple frames of fingerprint feature data corresponding to the predetermined region, and the heart rate information is determined according to the heart rate data.

Specifically, an average value of the each frame of fingerprint feature data may be firstly calculated to obtain the heart rate data, then a filtering operation is performed on the obtained heart rate data to remove noise, and finally the heart rate information is determined according to the heart rate data upon filtering. The details may be referenced to related disclosure in the above embodiment.

In addition, this embodiment may be incorporated with the above embodiment. For example, all the sensing units of the sensing array may be firstly scanned to collect one frame of fingerprint feature data for identity authentication, and then the sensing units in the predetermined region of the sensing array are scanned for multiple times to quickly collect multiple frames of fingerprint feature data for determination of accurate heart rate information.

It should be noted that, in the embodiments of the present application, the heart rate information is only described as an example, and when the vital sign information is other feature information, for example, blood oxygen or the like, adaptive modifications may be made to the specific calculation method in the embodiments of the present application to achieve the corresponding objective.

Embodiments of the present application further provide an electronic terminal. The electronic terminal includes the biological feature sensor as described above. The electronic terminal may be a mobile phone, a tablet computer, a computer or the like having the above biological feature sensor.

The apparatus according to the embodiments of the present application may be practiced by a computer program. A person skilled in the art should understand the above division of units and modules is only an exemplary one, and if the apparatus is divided into other units or modules or not divided, the technical solution shall also fall within the protection scope of the present application as long as the information object has the above functions.

A person skilled in the art shall understand that the embodiments of the present application may be described to illustrate methods, apparatuses (devices), or computer program products. Therefore, hardware embodiments, software embodiments, or hardware-plus-software embodiments may be used to illustrate the present application. In addition, the present application may further employ a computer program product which may be implemented by at least one non-transitory computer-readable storage medium with an executable program code stored thereon. The non-transitory computer-readable storage medium comprises but not limited to a disk memory, a CD-ROM, and an optical memory.

The present invention is described based on the flowcharts and/or block diagrams of the method, apparatus (device), and computer program product. It should be understood that each process and/or block in the flowcharts and/or block diagrams, and any combination of the processes and/or blocks in the flowcharts and/or block diagrams may be implemented using computer program instructions. These computer program instructions may be issued to a computer, a dedicated computer, an embedded processor, or processors of other programmable data processing device to generate a machine, which enables the computer or the processors of other programmable data processing devices to execute the instructions to implement an apparatus for implementing specific functions in at least one process in the flowcharts and/or at least one block in the block diagrams.

These computer program instructions may also be stored a non-transitory computer-readable memory capable of causing a computer or other programmable data processing devices to work in a specific mode, such that the instructions stored on the non-transitory computer-readable memory implement a product comprising an instruction apparatus, where the instruction apparatus implements specific functions in at least one process in the flowcharts and/or at least one block in the block diagrams.

These computer program instructions may also be stored on a computer or other programmable data processing devices, such that the computer or the other programmable data processing devices execute a series of operations or steps to implement processing of the computer. In this way, the instructions, when executed on the computer or the other programmable data processing devices, implement the specific functions in at least one process in the flowcharts and/or at least one block in the block diagrams.

Although the preferred embodiments of the present application are described above, once knowing the basic creative concept, a person skilled in the art can make other modifications and variations to these embodiments. Therefore, the appended claims are intended to be construed as covering the preferred embodiments and all the modifications and variations falling within the scope of the present application. Obviously, a person skilled in the art can make various modifications and variations to the present application without departing from the spirit and scope of the present application. In this way, the present application is intended to cover the modifications and variations if they fall within the scope of the appended claims of the present application and equivalent technologies thereof.

**Claims**

1. A vital sign information determination method, comprising:

   acquiring first biological feature data collected when an object presses on a biological feature sensor, the first biological feature data being used to reflect a body texture feature of the object;
   parsing a plurality of pieces of first biological feature data of the object to acquire second biological feature data, the second biological feature data being used to reflect a vital sign of a body of the object; and
   determining vital sign information of the body of the object according to the second biological feature data.

2. The method according to claim 1, wherein the acquiring first biological feature data collected when an object presses on a biological feature sensor comprises:
   acquiring the first biological feature data collected according to a predetermined collection rule when the object presses on the biological feature sensor, the predetermined collection rule being determined according to an arrangement manner of sensing arrays on the biological feature sensor.

3. The method according to claim 1, wherein the parsing a plurality of pieces of first biological feature data of the object to acquire second biological feature data comprises:
parsing the plurality of pieces of first biological feature data to remove texture information therefrom and acquire the second biological feature data.

4. The method according to claim 3, further comprising:

grouping the plurality of pieces of first biological feature data according to a predetermined collection rule to obtain several groups of first biological feature data; and correspondingly, the parsing the plurality of pieces of first biological feature data to remove texture information therefrom and acquire the second biological feature data comprises:
averaging a plurality of pieces of first biological feature data in different groups to remove the texture information therefrom and acquire a plurality of second biological feature data, wherein one group of first biological feature data corresponds to one second biological feature data.

5. The method according to claim 4, wherein the first biological feature data is one frame of the first biological feature data collected by performing collection for the sensing arrays on the biological feature sensor; the grouping the plurality of pieces of first biological feature data according to the predetermined collection rule comprises: using a plurality of pieces of first biological feature data obtained by performing a row scanning or a column scanning for the sensing arrays as a group of first biological feature data; or
when first biological feature data is multiple frames of the first biological feature data collected by performing scanning for the sensing arrays on the biological feature sensor, the grouping the plurality of pieces of first biological feature data comprises: using one frame of first biological feature data as a group of first biological feature data.

6. The method according to claim 5, wherein the determining vital sign information of the body of the object according to the second biological feature data comprises:
performing waveform peak statistical collection or spectrum analysis for a plurality of pieces of second biological feature data to determine the vital sign information of the body of the object.

7. The method according to claim 6, wherein the performing waveform peak statistical collection or spectrum analysis for a plurality of pieces of second biological feature data to determine the vital sign information of the body of the object comprises:

parsing the several groups of first biological feature data obtained by grouping one frame of the first biological feature data respectively to acquire the plurality of pieces of second biological feature data, and performing the waveform peak statistical collection or the spectrum analysis for the plurality of pieces of second biological feature data to determine the vital sign information of the body of the object; or
parsing the several groups of first biological feature data obtained by grouping multiple frames of the first biological feature data respectively to acquire the plurality of pieces of second biological feature data, and performing the waveform peak statistical collection or the spectrum analysis for the plurality of pieces of second biological feature data to determine the vital sign information of the body of the object

8. The method according to any one of claims 1 to 7, wherein the determining vital sign information of the body of the object according to the second biological feature data comprises:
performing filtering for the second biological feature data, and determine the vital sign information of the body of the object according to the second biological feature data upon the filtering.

9. The method according to claim 8, wherein the object is a finger or a palm, the first biological feature data is correspondingly fingerprint feature data or palmprint feature data, and the vital sign information is heart rate information.

10. The method according to claim 9, wherein the biological feature sensor comprises a light source, the light source shares an OLED light source in an OLED display screen of an electronic terminal where the biological feature sensor is configured.

11. An identity authentication method, comprising:

acquiring first biological feature data collected when an object presses on a biological feature sensor, and performing identity authentication for the object according to the first biological feature data, the first biological feature data being used to reflect a body texture feature of the object;
parsing a plurality of pieces of first biological feature data of the object to acquire second biological feature data, determining vital sign information of the body of the object according to the second biological feature data, and performing aliveness identification for the object according to the vital sign information, the second biological feature data being used to reflect a vital sign of a body of the object; and

judging whether the identity authentication is successful according to a result of the identity authentication and a result of the aliveness identification.

**12.** The method according to claim 11, wherein the performing aliveness identification for the object according to the vital sign information comprises:

comparing the vital sign information with reference vital sign information to perform aliveness identification for the object.

**13.** The method according to claim 11, wherein the parsing a plurality of pieces of first biological feature data of the object to acquire second biological feature data comprises:

parsing the plurality of pieces of first biological feature data to remove texture information therefrom and acquire the second biological feature data.

**14.** The method according to claim 13, further comprising:

grouping the plurality of pieces of first biological feature data according to a predetermined collection rule to obtain several groups of first biological feature data; and correspondingly, the parsing the plurality of pieces of first biological feature data to remove texture information therefrom and acquire the second biological feature data comprises:

averaging a plurality of pieces of first biological feature data in different groups to remove the texture information therefrom and acquire a plurality of second biological feature data, wherein one group of the first biological feature data corresponds to one second biological feature data.

**15.** The method according to claim 14, wherein the first biological feature data is one frame of the first biological feature data collected from the sensing arrays on the biological feature sensor; the grouping the plurality of pieces of first biological feature data according to the predetermined collection rule comprises: using a plurality of pieces of first biological feature data obtained by performing a row scanning or a column scanning for the sensing arrays as a group of first biological feature data; or

the first biological feature data is multiple frames of the first biological feature data collected by performing scanning for the sensing arrays on the biological feature sensor, the grouping the plurality of pieces of first biological feature data comprises: using one frame of the biological feature data as a group of first biological feature data.

**16.** The method according to claim 15, wherein the determining vital sign information of the body of the object according to the second biological feature data comprises:

performing waveform peak statistical collection or spectrum analysis for a plurality of pieces of second biological feature data to determine the vital sign information of the body of the object.

**17.** A biological feature sensor, comprising: a light source, a sensing array, a biological feature chip; wherein light emitted by the light source is reflected by an object and received by the sensing array to generate first biological feature data; the biological feature chip is configured to acquire the first biological feature data collected when the object presses on the biological feature sensor; the first biological feature data is parsed to determine vital signal information of a body of the object; wherein the first biological feature data is used to reflect a body texture feature of the object, and the second biological feature data is used to reflect a vital sign of the body of the object.

**18.** The biological feature sensor according to claim 17, wherein the light source shares an OLED light source of an OLED display screen of an electronic terminal where the biological feature sensor is configured, and/or the sensing array is arranged below the OLED display screen.

**19.** An electronic terminal, comprising a biological feature sensor as defined in claim 17 or 18.

| Acquire first biological feature collected when an object presses on a biological feature sensor | S11 |

| Parse a plurality of first biological feature data of the object to acquire second biological feature data | S12 |

| Determine vital sign information of the body of the object according to the second biological feature data | S13 |

FIG. 1

FIG. 2a

FIG. 2b

FIG. 3

FIG. 4

Acquire one frame of fingerprint feature data collected according to a predetermined collection rule when a finger presses on a biological feature sensor, and perform identity authentication according to the fingerprint feature data ⟩ S51

Parse one frame of fingerprint feature data of the finger to acquire heart rate data of a body of the finger, determine heart rate information of the body of the finger according to the heart rate data, and perform aliveness identification for the finger according to the heart rate information ⟩ S52

S53

Judge whether the identity authentication is successful according to a result of the identity authentication and a result of the aliveness identification

FIG. 5a

Row1
Row2

RowM

FIG. 5b

| Press a finger under test on a fingerprint identification region of an optical fingerprint sensor | S61 |

| Scan a predetermined region of an optical fingerprint sensor array for multiple times for data collection in multiple cycles to acquire multiple frames of fingerprint feature data corresponding to several sensing units in the predetermined region | S62 |

| Determine the heart rate data according to the multiple frames of fingerprint feature data corresponding to the predetermined region, and determine the heart rate information is determined according to the heart rate data | S63 |

FIG. 6a

Predetermined
Region

FIG. 6b

## INTERNATIONAL SEARCH REPORT

| International application No. |
| --- |
| **PCT/CN2017/082917** |

| A. | CLASSIFICATION OF SUBJECT MATTER |
| --- | --- |
| | G06K 9/00(2006.01)i |

According to International Patent Classification (IPC) or to both national classification and IPC

| B. | FIELDS SEARCHED |
| --- | --- |

Minimum documentation searched (classification system followed by classification symbols)

G06K 9/-

Documentation searched other than minimum documentation to the extent that such documents are included in the fields searched

Electronic data base consulted during the international search (name of data base and, where practicable, search terms used)

CNABS, CPRSABS, DWPI: 生命体征, 心跳, 脉搏, 特征, 指纹, 采集, 触摸, 触压, 解析, 传感器, 阵列, 纹理, vital signs, heartbeat, pulse, characteristic, fingerprint, collect+, touch, press, parse, sensor, array, texture

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| X | CN 105205464 A (YULONG COMPUTER TELECOMMUNICATION SCIENTIFIC (SHENZHEN) CO., LTD.) 30 December 2015 (2015-12-30) description, paragraphs 5-22 | 1-3，8-13，17-19 |
| A | CN 106462765 A (SHENZHEN GOODIX TECHNOLOGY CO., LTD.) 22 February 2017 (2017-02-22) entire document | 1-19 |
| A | CN 106473711 A (LENOVO MOBILE COMMUNICATION TECHNOLOGY LTD.) 08 March 2017 (2017-03-08) entire document | 1-19 |
| A | CN 104102925 A (ZHONGSHAN PINHUI INNOVATIVE PATENTED TECHNOLOGY DEVELOPMENT CO., LTD.) 15 October 2014 (2014-10-15) entire document | 1-19 |

☐ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 February 2018** | **09 February 2018** |

| Name and mailing address of the ISA/CN | Authorized officer |
| --- | --- |
| **State Intellectual Property Office of the P. R. China** **No. 6, Xitucheng Road, Jimenqiao Haidian District, Beijing 100088** **China** | |
| Facsimile No. **(86-10) 62019451** | Telephone No. |

Form PCT/ISA/210 (second sheet) (July 2009)

**INTERNATIONAL SEARCH REPORT**
Information on patent family members

International application No.

**PCT/CN2017/082917**

| Patent document cited in search report | | | Publication date (day/month/year) | Patent family member(s) | | | Publication date (day/month/year) |
|---|---|---|---|---|---|---|---|
| CN | 105205464 | A | 30 December 2015 | None | | | |
| CN | 106462765 | A | 22 February 2017 | WO | 2016074638 | A1 | 19 May 2016 |
| | | | | EP | 3218849 | A4 | 17 January 2018 |
| | | | | KR | 20160144453 | A | 16 December 2016 |
| | | | | EP | 3218849 | A1 | 20 September 2017 |
| | | | | US | 2016132712 | A1 | 12 May 2016 |
| | | | | IN | 201627038418 | A | 09 December 2016 |
| CN | 106473711 | A | 08 March 2017 | None | | | |
| CN | 104102925 | A | 15 October 2014 | None | | | |

Form PCT/ISA/210 (patent family annex) (July 2009)